# EUROPEAN PATENT APPLICATION

(11) **EP 3 633 376 A1**
(43) Date of publication of application: **08.04.2020**
(21) Application number: 17911553.0
(22) Date of filing: 02.06.2017
(51) Int. Cl.: G01N 33/574, C07K 16/28

(54) **PROTEIN MARKER FOR DIAGNOSING PROSTATE CANCER, METHOD FOR MEASURING THE QUANTITY OF A MARKER AND ALGORITHM FOR INTERPRETING THE RESULT**

(71) Applicant: Limited Liability Company "Prostagnost", Moscow 143026 (RU)
(72) Inventor: ZEMSKOVA, Marina Yurievna, Moskovskaya obl. 142290 (RU)
(74) Representative: Berggren Oy, Helsinki & Oulu
(86) International application number: PCT/RU2017/000386
(87) International publication number: WO 2018/222069

(57) **Abstract**

The invention relates to the field of biology and medicine, specifically to oncology, and can be used for diagnosing prostate cancer. For this purpose, use is made of a novel prostate cancer marker, which can be identified in the urine of a patient and constitutes of integrin-alpha V level or a fragment thereof. A method of diagnosing prostate cancer, comprising identifying integrin-alpha V in the urine of a patient, and a kit for carrying out the diagnosis, comprising at least one antibody specific to integrin-alpha V, or a functional fragment thereof, are also proposed. The invention makes it possible to carry out a non-invasive diagnosis of prostate cancer using a simple, sensitive and reliable method, which can be used under standard clinical laboratory conditions in inpatient or outpatient medical institutions.

## Description

### FIELD OF THE INVENTION

The invention relates to the field of biology and medicine, particularly, oncology, and can be used for diagnosis of prostate cancer

### BACKGROUND OF THE INVENTION

After skin cancer, prostate cancer (PC) is the most commonly diagnosed form of cancer. In many countries, PC is the second / third most common cause of death for men over the age of 40 years. Currently it is believed that early diagnosis is the most important factor in the fight against PC.

The traditional and most common approach of detecting PC is the PSA test (prostate specific antigen, PSA) in serum (Stamey TA, Yang N, Hay AR, McNeal JE, Freiha FS, Redwine E. Prostate-specific antigen as a serum marker for adenocarcinoma of the prostate. N Engl J Med. 1987 Oct 8; 317(15):909-16). However, during over 30 years of clinical practice a large amount of statistical data has been accumulated, indicating a low serum PSA specificity as a tumor marker, which manifests itself in the form of a significant number of false-positive results. Therefore, many researchers have focused on finding new tumor markers.

Two different PC diagnostic method can be sought-after in clinical practice: first is non-invasive fast simple method realizable with standard clinical laboratory equipment and eligible for routine / sequential analysis during clinical examination, monitoring the course of the disease, monitoring the effectiveness of therapy, etc.; second method is necessary for the final differential analysis, for which the only important parameter is closeness of accuracy to 100%.

Currently, a number of prostate cancer biomarkers (for example PCA3, TMPRSS2: ERG, PSCA (prostate stem cell antigen), AMCAR) that are determined by non-invasive techniques that measure the quantities of these markers in urine samples (see, for example, Koo K. M. et al., Colorimetric TMPRSS2-ERG Gene Fusion Detection in Prostate Cancer Urinary Samples via Recombinase Polymerase Amplification // Theranostics. 2016 Jun 15; 6 (9): 1415-24; Deras I. L. et al., PCA3: a molecular urine assay for predicting biopsy outcome // J Urol. 2008 Apr; 179 (4): 1587-92) are being actively studied. However, these markers require for their detection use of rather expensive methods based on polymerase chain reaction and/or immunohistochemistry, and these analyses are difficult to produce in the framework of a standard clinical laboratory.

Recently, the possibility of diagnosing prostate cancer using biomarkers of urine exosomes has been actively investigated. For example, patent US 7897356 B2 discloses a method for diagnosing prostate cancer by identifying a biosignal of exosomes, comprising capturing and isolating exosomes using common surface exosome proteins CD63, CD9 or CD81, and then performing a qualitative or quantitative analysis of the level of specific PC markers PSMA, PCSA, EpCam, B7H3 (CD276). Another proposed method and a kit for quantifying and determining exosomes for diagnosing prostate cancer and prostate hyperplasia (US 20130196355 A1) is based on an enzyme immunoassay using anti-Rab5 as a trapping antibody to isolate all the exosomes, from which subsequently exosomes are isolated using anti-PSA detecting antibodies and the level of these exosomes is compared to the reference level. However, firstly, a fast and non-invasive biomarker suitable for implementation of clinical laboratory diagnostics of prostate cancer on a standard laboratory equipment was never found; and secondly, isolation of exosomes, which is necessary for realization of the mentioned methods, is a very nontrivial task that cannot be adequately solved by majority of clinical laboratories.

Thus, despite existing studies on the diagnosis of prostate cancer, medical practice of population surveys and also monitoring risk groups, monitoring relapses, monitoring the course of the disease, monitoring the effectiveness of therapy still needs a new routine method that can be performed using common in clinical laboratories equipment.

### SUMMARY OF THE INVENTION

The task and the technical result of the invention is to find a new diagnostic marker for PC, allowing for a non-invasive diagnosis of the disease, as well as developing on its basis a simple, sensitive, reliable method for diagnosis of PC suitable for conventional clinical laboratories in hospitals and medical institutions.

The task is solved using a new composition for the diagnosis of prostate cancer, namely integrin alpha V (alone or in combination with other markers) or a fragment thereof detected in human subject's whole urine. According to the invention, the integrin alpha V or its fragment can be used as a biomarker by identifying them as an individual subunit and as a part of integrin heterodimers, such as αvβ1, αvβ3, αvβ5, αvβ6 and / or αvβ8.

The problem is also solved by a noninvasive method for detecting integrin alpha V or a fragment thereof in a human subject's whole urine for diagnosing prostate cancer.

In some implementations of the method, the level of integrin alpha V is determined as a level of an individual subunit or as a level of one of the integrin heterodimers selected from the following group: αvβ1, αvβ3, αvβ5, αvβ6, αvβ8.

If the detected concentrations of the integrin alpha V or its fragment in human subject's whole urine are above than 20 nanograms per milliliter then the absence of PC may be diagnosed.

In some implementations of the method, the method is performed together with additional clinical, laboratory or instrumental examinations.

In some particular realizations of the invention additional clinical, laboratory and/or instrumental examinations are performed if the concentration of the integrin alpha V or its fragment in patient's whole urine is 20 nanograms per milliliter or less.

The problem is also solved by a diagnostic kit for performing detection of integrin alpha V or a fragment thereof in a human subject's whole urine, comprising at least one antibody or its antigen-recognizing part that specifically binds to integrin alpha V or its fragments and not cross-specific to other urine peptides.

In some realizations of the invention, the kit includes a test system for conducting an enzyme-linked immunosorbent assay.

### BRIEF DESCRIPTION OF THE DRAWINGS

Fig. 1. The results of the integrin alpha V level determination in the first portions of morning urine samples taken from 50 men older than 50:
   n - number of measurements. Horizontal line - 20 ng/ml of the biomarker.
Fig. 2. The results of mathematical processing of the data shown in Fig. 1.
   X axis represents the relative level of the integrin alpha V, ng/ml
   Y-axis represents is the probability of detecting integrin alpha V in the urine of various groups of subjects. Vertical lines show mathematical expectations.

### TERMS AND DEFINITIONS

Herein this description the terms "includes" and "including" shall be construed as "includes, among other things." These terms are not intended to be interpreted as "consists only of."

The term PC refers to prostate cancer, BPH refers to benign prostate formations (adenoma, prostatitis) and "normal samples" refers to samples obtained from individuals over 50 years old without pathologies of the prostate gland.

The term "whole urine" refers to both urine samples that are not subjected to any processing and samples obtained after centrifugation, filtration, or other procedures that allow separation of soluble urine components from the insoluble fraction.

"Marker" or "biomarker", unless otherwise specified, in the present description means the following: integrin alpha V (identification number UniProtKB P06756-1; and its isoforms (P06756-2; P06756-3). Integrin alpha V (other names αV, CD51, MSK8, vitronectin receptor α (VNRα)) is a membrane protein, a glycoprotein from the integrins superfamily, the ITGaV gene product located on the 2nd chromosome at position 2q31-q32.

As used herein, the term "antibody" refers to a whole immunoglobulin or its fragment, for example, of the Fab type or (Fab')₂ which retains antigen-binding activity. It may be a naturally occurring antibody or an antibody obtained by immunization (including, for example, a chimeric antibody or a humanized antibody) or a recombinant antibody constructed using genetic engineering techniques.

In the present description, an antibody or its fragment means an antibody that specifically binds to alpha V integrin or its fragment and does not possess cross-specificity to other proteins (peptides, their fragments) of urine, i.e. is an antibody that has (significantly) greater affinity for integrin alpha V or its fragment than for other proteins (peptides, their fragments) of urine.

In the diagnostic kit of the present invention, antibodies obtainable from commercial suppliers can be used, or these antibodies can be obtained by the above mentioned methods or other means.

Without further clarification, it is assumed that on the basis of the above description, an ordinary specialist qualified in this field is able to apply the present invention in its entirety. The following options are illustrative only. They in no way limit the disclosure of the present invention. All cited publications are included as references.

The possibility of an objective manifestation of the technical result using the invention is confirmed by reliable data given in the examples containing experimental information obtained in the process of conducting research according to the methods adopted in this field. The invention is illustrated by figures.

The following examples are given for demonstration of the method according to this invention and should not be treated as limiting the invention in any way.

### DETAILED DESCRIPTION OF THE INVENTION

The integrin alpha V (HGNC: 6150; Entrez Gene: 3685; Ensembl: ENSG00000138448; OMIM: 193210; UniProtKB: P06756) is a membrane protein, a glycoprotein from the integrins superfamily, the ITGaV gene product located on the 2nd chromosome at position 2q31-q32. Integrin alpha V consists of 1048 amino acids. The mature integrin alpha V molecule is a glycoprotein with a length of 1018 amino acids (see SEQ NO: 1), the molecular weight of the protein part is 116.0 kDa.

A mature integrin alpha V molecule forms a heterodimeric complex with beta-1, beta-3, beta-5, beta-6 or beta-8 integrins. The alpha V integrins are receptors for a number of ligands, recognizing the sequence of arginine-glycine-aspartate (R-G-D). Integrins with the alpha V subunit are involved in many processes of embryo development, in angiogenesis and osteoporosis.

In the course of the research it was unexpectedly discovered that the quantitative content (level) of integrin alpha V in whole urine correlates with PC, which allows it to be used as a biomarker for the diagnosis of PC, and also made it possible to develop a new method for the diagnosis of PC on the basis of this new biomarker. Quantitative determination of integrin alpha V in whole urine allows non-invasive diagnosis of the disease, and a simple analysis technique allows the proposed method to be used in a standard clinical laboratory of hospitals or medical institutions.

The invention also covers all kinds of kits and methods for diagnosing prostate cancer. Such kits may include antibodies against integrin alpha V or its fragments, which can be detected both as the individual subunit and / or as a part of heterodimers, such as heterodimeric complexes with beta-1, beta-3, beta-5, beta-6, and / or beta 8. According to the invention any antibody or its fragment, that specifically bind to alpha V integrin and are not cross-specific to other urine peptides can be used. Such antibodies may include, but are not limited to, any antibody / antibody fragments against integrin alpha V known in the prior art (for example, Polyclonal Antibody to Integrin Alpha V (ITGaV) http://www.elabscience.com/Manual/Antibody/EN/ENT2365.pdf, http://cloud-clone.com/products/PAB282Hu01.html, http://www.genetex.com/Integrin-alpha-5-antibody-C-term-GTX81964.html, Monoclonal Antibody to Integrin Alpha V (ITGaV) http://www.abcam.com/integrin-alpha-5-antibody-epr7854-ab150361.html, http://www.sigmaaldrich.com/catalog/product/mm/mabt207, http://cloud-clone.com/products/MAB282Hu22.html, http://www.antibodies-online.com/abstract/Integrin+alpha+V+(ITGAV)+Antibody/) and new, previously unknown antibodies / antibody fragments, including antibodies (or antibody fragments), modified to increase their affinity by known methods, which can be obtained using known methods, such as an immunization method (including, for example, chimeric antibodies or humanized antibodies) or a method for producing recombinant antibodies constructed with using genetic engineering techniques.

The diagnostic kit includes at least one antibody / antibody fragment, specifically binding to integrin alpha V (for example, either immobilized on a substrate, or in a lyophilized form, or as an aqueous solution) and one or more reagents necessary for diagnostic analysis. If an anti-integrin alpha V antibody is labeled with an enzyme, the kit may include substrates and cofactors required for the enzyme activity detection (for example, a precursor substrate that provides a detectable chromophore or fluorophore). In addition, other additives may be included in the kit, such as stabilizers, buffers (for example, blocking buffer) and others. In certain realizations an anti-integrin alpha V antibody included in the diagnostic kit is immobilized on a solid surface or a granular material (for example, a glass slide or plastic plate or a diagnostic strip, etc.). The relative amounts of different reagents can vary widely to ensure concentrations of reagents in the solution that can optimize sensitivity of the assay. In a specific realization an antibody and one or more reagents may be provided (individually or jointly) in the form of dry powders, usually lyophilized, including excipients, which will produce a reagent solution having a suitable concentration if dissolved.

In some realizations, a set for the diagnosis of PC may be a test system of arbitrary design, for example, diagnostic strips, an array, such as a protein microarray, a chip, etc., or a set of reagents for use in manual or automated analysis, or with a partially automatic / fully automatic / robotic analyzer. The reagent kit may also include an IVD device, such as a plate, with immobilized antibodies, which allows for conducting a solid phase ELISA or any other assay with a sensitivity of at least 0.2 ng / ml.

In some realizations of the invention other known from the prior art clinical, laboratory and / or instrumental methods of prostate gland examination may additionally be applied.

The genuine ITGaV biomarker is measured in whole urine or any other urine fraction, including exosomes, but not limited to them.

In some realizations of the invention, the integrin alpha V biomarker can be used in the PC diagnosis in combination with any other biomarkers that might improve the quality (reliability, accuracy, etc.) of the PC diagnosis. These biomarkers include, but are not limited to prostate-specific antigen, PCA3, TMPRSS2:ERG, PSCA (prostate stem cell antigen), AMCAR, and / or others.

### EXAMPLE IMPLEMENTATION OF THE INVENTION

Diagnosis of PC can be carried out by any method of analysis that is able to determine the level of integrin alpha V in the patient's urine. In one particular case, the diagnosis was carried out as follows:
- about 30 ml (up to 50 ml is allowed) of the first portion of morning urine was taken from a subject;

The following procedures were used to determine the integrin alpha V level in urine:
- the urine sample was clarified by centrifugation for 15 minutes at a speed of 2,000 rpm (filtration through membranes with a pore size of 0.22-0.45 nm or any other available method can be used to separate the soluble fraction of urine proteins from the insoluble sediment);
- the level of integrin alpha V was determined in this particular case by ELISA using commercially available kit "SEB282Hu 96 Tests. Enzyme-linked Immunosorbent Assay Kit for Integrin Alpha V (ITGaV)" from Cloud-Clone Corp. (http://cloud-clone.com/products/SEB282Hu.html), by strictly following the instructions (http://cloud-clone.com/manual/ELISA-Kit-for-Integrin-Alpha-V-(ITGaV)-SEB282Hu.pdf).

Briefly:
- 25 µl. of urine was introduced into a well of a 96-well plate with immobilized antibodies;
- incubated for 1.5 hours at 37 °C;
- the liquid was removed and 100 µl of the detection reagent A were added;
- incubated for 1.5 hours at 37 °C;
- the liquid was removed and the well was washed 3 times with the washing solution. After each wash, the liquid was carefully removed by shaking out on a filter paper;
- 100 µl of the detection reagent B were added;
- the plate was incubated for 0.5 hours at 37 °C;
- the liquid was removed, and the well was washed 5 times with the washing solution. After each wash, the liquid was carefully removed by shaking out on a filter paper;
- 100 µl of TMB substrate were added;
- incubated for 20 min at 37 °C;
- 50 µl of the stop reagent were added;
- the absorbance was measured immediately photometrically at 450 nm. To determine the level of ITGaV in each experimental urine sample according to the manufacturer's instructions, a calibration curve obtained from a separate measurement of various dilutions of a pure (homogeneous) protein was used.

At the same time, an increase in the accuracy of analysis and reproducibility of results can be obtained in two ways:
- due to control of the process of collecting urine: one needs exactly 30-50 ml of the first portion of morning urine;
- collecting 30-50 ml of the first portion of urine after a prostate massage.

Urine can be stored at + 4 °C for a day and for 2-3 weeks at -18 °C.

In some cases, it is also advisable to repeat the analysis.

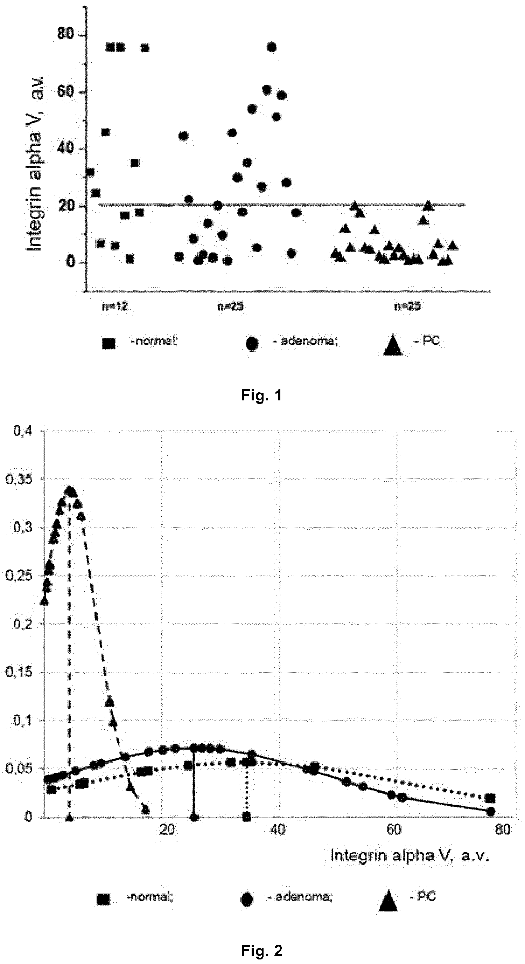

Fig.1 shows the experimental results of measuring the level of integrin alpha V in the first portion of morning urine of three groups of Caucasians with age over 50 years: healthy people, patients with benign prostatic hyperplasia and patients with prostate cancer. The urine samples were obtained by the above method.

The graph of Fig. 2 shows the curves of the distribution of values for the three data sets and the mathematical expectations of the corresponding measurements (vertical lines). As follows from the graph, the mathematical expectations of the biomarker level in healthy people and patients with benign hyperplasia (from 24 ng/ml to 32 ng/ml) differ significantly from the mathematical expectation of the biomarker level in patients with prostate cancer (∼4 ng/ml). Mathematical processing was carried out under the assumption of a Gaussian distribution of the original data.

As follows from the obtained data concentration of the integrin alpha V in the urine over 20 ng/ml evidence for absence of PC (100%), wherein patient may be healthy or have prostatic hyperplasia (prostatitis/adenoma); the level of integrin alpha V below 20 ng/ml should lead to an additional examination of a subject, for example, by means of biopsy and histological analysis.

The given data indicates that the integrin alpha V may be used as a new biomarker for PC diagnosis, its level determination in the urine may be used for noninvasive PC diagnosis and for developing on its basis of a simple sensitive reliable and implementable in the framework of a standard clinical laboratories in hospitals and medical institutions method for PC diagnosis.

Although the invention has been described with a reference to the disclosed realizations, it should be obvious to those skilled in the art that the specific experiments described in detail here are given only for the purpose of illustrating the present invention and should not be construed as limiting the scope of the invention in any way. It should be clear that it is possible to implement various modifications without departing from the gist of the present invention.

## Claims

1. A composition for the diagnosis of prostate cancer, comprising integrin alpha V or a fragment thereof used as a marker detected in whole urine.

2. A noninvasive method for detecting integrin alpha V or a fragment thereof in a human subject's whole urine for diagnosing prostate cancer.

3. The noninvasive method according to claim 2, wherein the level of integrin alpha V is determined as a level of an individual subunit or as a level of one of the integrin heterodimers selected from the following group: αvβ1, αvβ3, αvβ5, αvβ6, αvβ8.

4. The method according to any one of claims 2 or 3, wherein the human subject is detected with the integrin alpha V level higher than 20 ng / ml indicating an absence of prostate cancer.

5. The method according to claim 2, wherein the method is used together with additional clinical, laboratory or instrumental examinations.

6. The method according to claim 5, where additional clinical, laboratory or instrumental examinations are conducted when the level of integrin alpha V or a fragment thereof is lower than 20 ng / ml.

7. A diagnostic kit for performing detection of integrin alpha V or a fragment thereof in a human subject's whole urine, comprising at least one antibody or its antigen-recognizing part that specifically binds to integrin alpha V or its fragments.

8. The diagnostic kit according to claim 7, where the kit includes a test system for conducting an enzyme-linked immunosorbent assay.


SEQUENCE LISTING

<110> LIMITED LIABILITY COMPANY "PROSTAGNOST" [RU/RU]

<120> PROTEIN MARKER FOR DIAGNOSING PROSTATE CANCER, METHOD FOR MEASURING
THE QUANTITY OF A MARKER AND ALGORITHM FOR INTERPRETING THE RESULT

<150> PCT/RU2017/000386
<151> 2017-06-02

<160> 1

<210> 1
<211> 1018
<212> PRT
<213> Homo sapiens

<400> 1
Phe Asn Leu Asp Val Asp Ser Pro Ala Glu Tyr Ser Gly Pro Glu Gly
1               5                   10                  15
Ser Tyr Phe Gly Phe Ala Val Asp Phe Phe Val Pro Ser Ala Ser Ser
            20                  25                  30
Arg Met Phe Leu Leu Val Gly Ala Pro Lys Ala Asn Thr Thr Gln Pro
        35                  40                  45
Gly Ile Val Glu Gly Gly Gln Val Leu Lys Cys Asp Trp Ser Ser Thr
    50                  55                  60
Arg Arg Cys Gln Pro Ile Glu Phe Asp Ala Thr Gly Asn Arg Asp Tyr
65                  70                  75                  80
Ala Lys Asp Asp Pro Leu Glu Phe Lys Ser His Gln Trp Phe Gly Ala
                85                  90                  95
Ser Val Arg Ser Lys Gln Asp Lys Ile Leu Ala Cys Ala Pro Leu Tyr
            100                 105                 110
His Trp Arg Thr Glu Met Lys Gln Glu Arg Glu Pro Val Gly Thr Cys
        115                 120                 125
Phe Leu Gln Asp Gly Thr Lys Thr Val Glu Tyr Ala Pro Cys Arg Ser
    130                 135                 140
Gln Asp Ile Asp Ala Asp Gly Gln Gly Phe Cys Gln Gly Gly Phe Ser
145                 150                 155                 160
Ile Asp Phe Thr Lys Ala Asp Arg Val Leu Leu Gly Gly Pro Gly Ser
                165                 170                 175
Phe Tyr Trp Gln Gly Gln Leu Ile Ser Asp Gln Val Ala Glu Ile Val
            180                 185                 190
Ser Lys Tyr Asp Pro Asn Val Tyr Ser Ile Lys Tyr Asn Asn Gln Leu
        195                 200                 205
Ala Thr Arg Thr Ala Gln Ala Ile Phe Asp Asp Ser Tyr Leu Gly Tyr
    210                 215                 220
Ser Val Ala Val Gly Asp Phe Asn Gly Asp Gly Ile Asp Asp Phe Val
225                 230                 235                 240
Ser Gly Val Pro Arg Ala Ala Arg Thr Leu Gly Met Val Tyr Ile Tyr
                245                 250                 255
Asp Gly Lys Asn Met Ser Ser Leu Tyr Asn Phe Thr Gly Glu Gln Met
            260                 265                 270
Ala Ala Tyr Phe Gly Phe Ser Val Ala Ala Thr Asp Ile Asn Gly Asp
        275                 280                 285
Asp Tyr Ala Asp Val Phe Ile Gly Ala Pro Leu Phe Met Asp Arg Gly
    290                 295                 300
Ser Asp Gly Lys Leu Gln Glu Val Gly Gln Val Ser Val Ser Leu Gln
305                 310                 315                 320
Arg Ala Ser Gly Asp Phe Gln Thr Thr Lys Leu Asn Gly Phe Glu Val
                325                 330                 335
Phe Ala Arg Phe Gly Ser Ala Ile Ala Pro Leu Gly Asp Leu Asp Gln
            340                 345                 350
Asp Gly Phe Asn Asp Ile Ala Ile Ala Ala Pro Tyr Gly Gly Glu Asp

        355                 360                 365
Lys Lys Gly Ile Val Tyr Ile Phe Asn Gly Arg Ser Thr Gly Leu Asn
    370                 375                 380
Ala Val Pro Ser Gln Ile Leu Glu Gly Gln Trp Ala Ala Arg Ser Met
385                 390                 395                 400
Pro Pro Ser Phe Gly Tyr Ser Met Lys Gly Ala Thr Asp Ile Asp Lys
                405                 410                 415
Asn Gly Tyr Pro Asp Leu Ile Val Gly Ala Phe Gly Val Asp Arg Ala
            420                 425                 430
Ile Leu Tyr Arg Ala Arg Pro Val Ile Thr Val Asn Ala Gly Leu Glu
        435                 440                 445
Val Tyr Pro Ser Ile Leu Asn Gln Asp Asn Lys Thr Cys Ser Leu Pro
    450                 455                 460
Gly Thr Ala Leu Lys Val Ser Cys Phe Asn Val Arg Phe Cys Leu Lys
465                 470                 475                 480
Ala Asp Gly Lys Gly Val Leu Pro Arg Lys Leu Asn Phe Gln Val Glu
                485                 490                 495
Leu Leu Leu Asp Lys Leu Lys Gln Lys Gly Ala Ile Arg Arg Ala Leu
            500                 505                 510
Phe Leu Tyr Ser Arg Ser Pro Ser His Ser Lys Asn Met Thr Ile Ser
        515                 520                 525
Arg Gly Gly Leu Met Gln Cys Glu Glu Leu Ile Ala Tyr Leu Arg Asp
    530                 535                 540
Glu Ser Glu Phe Arg Asp Lys Leu Thr Pro Ile Thr Ile Phe Met Glu
545                 550                 555                 560
Tyr Arg Leu Asp Tyr Arg Thr Ala Ala Asp Thr Thr Gly Leu Gln Pro
                565                 570                 575
Ile Leu Asn Gln Phe Thr Pro Ala Asn Ile Ser Arg Gln Ala His Ile
            580                 585                 590
Leu Leu Asp Cys Gly Glu Asp Asn Val Cys Lys Pro Lys Leu Glu Val
        595                 600                 605
Ser Val Asp Ser Asp Gln Lys Lys Ile Tyr Ile Gly Asp Asp Asn Pro
    610                 615                 620
Leu Thr Leu Ile Val Lys Ala Gln Asn Gln Gly Glu Gly Ala Tyr Glu
625                 630                 635                 640
Ala Glu Leu Ile Val Ser Ile Pro Leu Gln Ala Asp Phe Ile Gly Val
                645                 650                 655
Val Arg Asn Asn Glu Ala Leu Ala Arg Leu Ser Cys Ala Phe Lys Thr
            660                 665                 670
Glu Asn Gln Thr Arg Gln Val Val Cys Asp Leu Gly Asn Pro Met Lys
        675                 680                 685
Ala Gly Thr Gln Leu Leu Ala Gly Leu Arg Phe Ser Val His Gln Gln
    690                 695                 700
Ser Glu Met Asp Thr Ser Val Lys Phe Asp Leu Gln Ile Gln Ser Ser
705                 710                 715                 720
Asn Leu Phe Asp Lys Val Ser Pro Val Val Ser His Lys Val Asp Leu
                725                 730                 735
Ala Val Leu Ala Ala Val Glu Ile Arg Gly Val Ser Ser Pro Asp His
            740                 745                 750
Val Phe Leu Pro Ile Pro Asn Trp Glu His Lys Glu Asn Pro Glu Thr
        755                 760                 765
Glu Glu Asp Val Gly Pro Val Val Gln His Ile Tyr Glu Leu Arg Asn
    770                 775                 780
Asn Gly Pro Ser Ser Phe Ser Lys Ala Met Leu His Leu Gln Trp Pro
785                 790                 795                 800
Tyr Lys Tyr Asn Asn Asn Thr Leu Leu Tyr Ile Leu His Tyr Asp Ile
                805                 810                 815
Asp Gly Pro Met Asn Cys Thr Ser Asp Met Glu Ile Asn Pro Leu Arg
            820                 825                 830
Ile Lys Ile Ser Ser Leu Gln Thr Thr Glu Lys Asn Asp Thr Val Ala
        835                 840                 845
Gly Gln Gly Glu Arg Asp His Leu Ile Thr Lys Arg Asp Leu Ala Leu
    850                 855                 860

Ser Glu Gly Asp Ile His Thr Leu Gly Cys Gly Val Ala Gln Cys Leu
865                 870                 875                 880
Lys Ile Val Cys Gln Val Gly Arg Leu Asp Arg Gly Lys Ser Ala Ile
                885                 890                 895
Leu Tyr Val Lys Ser Leu Leu Trp Thr Glu Thr Phe Met Asn Lys Glu
            900                 905                 910
Asn Gln Asn His Ser Tyr Ser Leu Lys Ser Ser Ala Ser Phe Asn Val
        915                 920                 925
Ile Glu Phe Pro Tyr Lys Asn Leu Pro Ile Glu Asp Ile Thr Asn Ser
    930                 935                 940
Thr Leu Val Thr Thr Asn Val Thr Trp Gly Ile Gln Pro Ala Pro Met
945                 950                 955                 960
Pro Val Pro Val Trp Val Ile Ile Leu Ala Val Leu Ala Gly Leu Leu
                965                 970                 975
Leu Leu Ala Val Leu Val Phe Val Met Tyr Arg Met Gly Phe Phe Lys
            980                 985                 990
Arg Val Arg Pro Pro Gln Glu Glu Gln Glu Arg Glu Gln Leu Gln Pro
        995                 1000                1005
His Glu Asn Gly Glu Gly Asn Ser Glu Thr
    1010                1015